Europäisches Patentamt

European Patent Office

Office européen des brevets

(11) Publication number: 0 379 765
A1

## EUROPEAN PATENT APPLICATION

(21) Application number: 89300818.5

(22) Date of filing: 27.01.89

(51) Int. Cl.5: **A61K 7/48, A61K 7/00**

(43) Date of publication of application:
01.08.90 Bulletin 90/31

(84) Designated Contracting States:
AT BE CH DE ES FR GB GR IT LI LU NL SE

(71) Applicant: VARIX AG
Aeulestrasse 38
FL-9490 Vaduz(LI)

(72) Inventor: Gonen, Shmuel
2 Tnust Hameri Street
Kirvat Ono(IL)
Inventor: Grossman, Shlomo
156 Snderot Hayeled
Ramat Gan(IL)

(74) Representative: Ellis-Jones, Patrick George
Armine et al
J.A. KEMP & CO. 14 South Square Gray's Inn
London WC1R 5EU(GB)

(54) Method and composition for promoting skin health.

(57) Improved skin health due to the improved respiratory function and/or metabolism of skin cells, is produced by applying to the skin of a living human a composition of matter (which is maintained under superatmospheric pressure in a pressurized container) comprising physical admixture of specified amounts of molecular oxygen with a dermatologically acceptable carrier. The carrier is restricted to a fluorocarbon-free carrier, except when the composition includes a propellant other than one comprising molecular oxygen. The invention also relates to the composition per se. In a preferred embodiment, the composition comprises molecular oxygen, dermatologically acceptable carrier, a propellant, and a foam stabilizer.

EP 0 379 765 A1

## METHOD AND COMPOSITION FOR PROMOTING SKIN HEALTH.

The present invention relates to a method for promoting skin health, due to the improved metabolism of skin cells, and in particular to the improved respiratory function thereof. The improved skin health may manifest itself, e.g., in a desirable skin cosmetic effect. The invention relates also to a composition of matter which may be used in this method.

Whatever the action of oxygen contained in the atmosphere on skin cells may be, this can be relevant to the outermost layer of cells only, since there is no evidence that such oxygen penetrates the outer surface of the skin. However, it is known that making oxygen available to cells of the skin layer generally, has a beneficial effect on the respiratory function of skin cells. The derived benefits of making oxygen available to cells of the skin layer generally include enhancement of the healing of wounds, as well as a desirable cosmetic effect. In the past, this desideratum has usually been achieved by means of activated oxygen (see e.g. French Patent Application No. 2,285,119 published in 1976). However, the disadvantage of this method, in which nascent oxygen is released from a chemical reaction, or oxygen is activated by radiation, is that the nascent or otherwise activated oxygen is likely to be reactive in situ and therefore liable to give rise to possibly irreversible oxidative damage to constituents of the skin cells.

More recently, it has been found that certain perfluoro compounds have an oxygen transport ability and can even perform the oxygen-carrying function of red blood cells; see e.g. Monograph No. 4090 in The Merck Index, 10th edn., 1983. The treatment of burn wounds with a liquid containing molecular oxygen and comprising a substantially fluorinated carbon material or a mono or dibrominated derivative thereof having an ability to transport oxygen, as a means of accelerating normal healing, is the subject of U.S. Patent No. 4,366,169 (White), which issued December 28, 1982. Prior to White, the beneficial effect of oxygen on the healing of burn wounds had been known; see e.g. Soviet Union Patent No. 628893 (published in 1978) in which a such wounds were treated with a detergent/disinfectant mixture foamed with oxygen at 2-5 atm.

The possibility of using a solution of oxygen in fluorocarbon derivatives in order to achieve a cosmetic effect was known as early as February 1976, from published French Patent Application No. 2,280,360. The solution was dispensed from an aerosol using oxygen as the propellant. A very recent Japanese Patent Publication, namely No. 63-91310 published April 22, 1988, proposes the use of a strongly magnetic material dissolved in a fluorocarbon solvent and mixed with oxygen, in order to achieve a cosmetic effect.

The present inventors have now discovered that when molecular oxygen is mixed with a suitable fluorocarbon-free carrier, and the mixture is applied to the skin, sufficient oxygen is retained in the mixture so that when the latter penetrates the skin, the molecular oxygen is carried with it and subsequently beneficially affects the metabolism, and in particular the respiration, of skin cells. This discovery may be regarded as a surprising one, since on general grounds it would be expected that the admixed oxygen would be rapidly released to the air, rather than being transmitted through the skin, particularly in view of the most recent published art which requires the use of a carrier comprising fluorocarbon solvent, presumably because of the known solubility of oxygen in perfluorocarbons.

It is therefore an object of the invention to provide a method by which molecular oxygen is carried through the outermost layer of skin in order to exert a beneficial effect on the cells of the skin layer generally. It is also an object of the invention to provide such a method which involves the physical release of oxygen in an admixture, and thereby avoids possible oxidative damage to cell constituents which could be caused by the use of agents which release oxygen by means of a chemical reaction. It is a further object of the invention to provide a method for producing promoting skin health, and more particularly a method for producing a desirable skin cosmetic effect.

It is yet a further object of the invention to provide a composition of matter which may be used in the method of the invention. A still further object of the invention is to provide a convenient form in which the composition of the invention may be applied in accordance with the method of the invention. Another object of the invention is to avoid the use of relatively expensive perfluorocarbons and other fluorinated derivatives, which are very largely untried, and would, if used, add an appreciable element of cost to the cosmetic preparations in question. Other objects of the invention will appear from the description which follows.

## SUMMARY OF THE INVENTION

The present invention accordingly provides in one embodiment a method for promoting skin health (and for cosmetically improving the skin), due to the improved respiratory function and/or metabolism of skin cells, which comprises applying to the skin of a living human a composition of matter which comprises a physical admixture of molecular oxygen with a dermatologically acceptable fluorocarbon-free carrier. The composition, which may be maintained under superatmospheric pressure in a pressurized container, also forms part of the present invention per se.

From the foregoing summary, it will be apparent that the expression "promoting skin health" includes also producing a beneficial effect on the skin and in particular improved respiratory function and/or metabolism of skin cells, and more especially a desirable cosmetic effect. Also, if the health of the skin is promoted by application of the composition of the invention, this will create a situation in which it is believed the healing of burns and other skin wounds will be enhanced.

## DETAILED DESCRIPTION OF THE INVENTION

The dermatologically acceptable fluorocarbon-free carrier used in the method and composition of the invention is preferably a cosmetically acceptable carrier, and it may be, for example, in the form of a liquid or semisolid, which is non-irritating and does not otherwise adversely affect the skin. It will be appreciated that whereas the use of fluorocarbon solvents in skin care products is uncommon and to the extent that they are used at all must still be regarded as in the very early experimental stages, the present invention is able to utilize carriers which have been known and used over a period of time. By way of example, any suitable cream or lotion may be utilized; thus, face creams, body lotions, hair creams or lotions, moisture creams, other skin conditioning creams, skin masks, liniments and shaving creams are contemplated for use as carriers in the method and composition of the invention. As an example of a dermatologically acceptable carrier which is not necessarily cosmetically acceptable, there may be cited petroleum jelly, which may be used as a carrier in accordance with the present invention.

It will be appreciated that in general the carrier may comprise at least one member selected from the group consisting of water and organic media such as (for example) ethanol, propanol, isopropanol, glycols (e.g. dipropylene glycol), esters (e.g. isopropyl myristate, isopropyl palmitate and/or diisopropyl adipate) and paraffin oil. In one embodiment, therefore, there may be used an aqueous carrier which comprises, in addition to water, at least one member selected from the group consisting of (for example) ethanol, propanol, isopropanol, glycols (e.g. dipropylene glycol), esters (e.g. isopropyl myristate, isopropyl palmitate and/or diisopropyl adipate) and paraffin oil. In another embodiment of the carrier, added water may be substantially absent, and the carrier will comprise at least one member selected from the group consisting of (for example) ethanol, propanol, isopropanol, glycols (e.g. dipropylene glycol), esters (e.g. isopropyl myristate, isopropyl palmitate and/or diisopropyl adipate) and paraffin oil.

While it is believed that the molecular oxygen, which is a principal ingredient of the admixture of the invention, is unlikely to have a deleterious oxidative effect on either the carrier or the cell constituents, nevertheless, if desired, an antioxidant may be present in the admixture as an added ingredient. Of course, commercially available creams and lotions will often contain antioxidants, and in this case further antioxidant may be optionally added, or in the case of a carrier not containing antioxidant this may in any case be added, if desired. Where an antioxidant is used, this may comprise, for example, at least one member selected from the group consisting of BHA (butylated hydroxy anisole), BHT (butylated hydroxy toluene), beta-carotene, propyl gallate and alpha-tocopherol. By way of example only, the carrier may contain from about 0.005 to about 5%, preferably from about 0.1 to about 1%, by weight, of antioxidant. The compositions may also contain e.g. preservatives (including antifungal preservatives); in this context, the parabens, i.e. the alkyl esters (such as the methyl, ethyl, propyl and butyl esters) of p-hydroxybenzoic acid may be utilized.

The admixture of molecular oxygen and carrier is preferably maintained under superatmospheric pressure prior to use, in order to prevent escape of oxygen during storage. Desirably, the admixture is applied to the skin from a pressurized container, such as an aerosol container which contains additionally a propellant. It is preferred that the aerosol container be well shaken before applying the admixture to the skin in accordance with the invention.

It is to be understood that in general, any types of aerosol containers, which are known to those skilled in the art, may be utilized in applying the present invention. Reference may be made, by way of example, to Sanders, Handbook of Aerosol Technology, Van Nostrand Reinhold Company, 2nd Edition 1979, the disclosures of which are incorporated herein by reference. On pages 74-77 of this book there are described the bag-in-can and the piston systems; it is believed that these systems may be especially useful in applying the present invention insofar as it could be possible to maintain a better constancy of the proportion of oxygen in the composition throughout the life of the aerosol, than when using other aerosol systems. However, this belief should not be construed as detracting from the utility of aerosols generally, in applying the present invention. In the bag-in-can systems, any suitable materials can of course be used for the bag; merely by way of example, Sanders (loc cit) mentions a blend of nylon and polyolefins, but more recently polyethylene, and even aluminum foil have been proposed for this purpose.

In accordance with a particular embodiment, the composition of the invention is generated in the form of a foam, the structure of which maintains an enriched microenvironment of oxygen in contact with the skin. More preferably, a foam stabilizer is incorporated in the composition. Thus according to an embodiment of the invention, there is provided a method for promoting skin health, which comprises applying to the skin of a living human a composition of matter which comprises a physical admixture of molecular oxygen with a dermatologically acceptable carrier, a propellant, and a foam stabilizer, which composition has been maintained under superatmospheric pressure in a pressurized container from which it is thus applied, the amount of molecular oxygen present in the admixture being not less than 0.25 wt. %, and provided that when the propellant comprises molecular oxygen, then the carrier is a fluorocarbon-free carrier.

Examples of foam stabilizers are soaps such as sodium stearate and sodium palmitate, synthetic soaps such as sodium cetyl sulfate, other surface active agents, glycerol, proteins such as gelatin, polymeric foam stabilizers such as methylcellulose and polyvinyl alcohol, other foam stabilizers such as saponins, and mixtures thereof. It will be appreciated that (e.g.) commercially available skin lotions may already contain substances such as surface active agents which may act as foam stabilizers. It will evidently be within the competence of a person with skill in the art, to determine whether further foam stabilizer needs to be added to a composition for treatment of the skin, what kind of foam stabilizer would be suitable, and the optimum proportion thereof.

The molecular oxygen utilized in accordance with the present invention is preferably substantially pure, although a gas which is enriched in its oxygen content, and which preferably contains at least a major amount of oxygen and trace amounts or at most minor amounts of other harmless gases may also be used. Air, which contains a relatively minor amount of oxygen, is impractical for the present purpose of providing a relatively high concentration in the composition of the invention, although as will be seen from the description below, air may be used for another purpose, i.e. as propellant. Molecular oxygen may be used to provide both the active ingredient of the composition as well as the propellant, except when a fluorocarbon carrier is utilized.

The propellant may in general be a pressurized gas which comprises at least one component selected from the group consisting of carbon dioxide, nitrous oxide, argon, nitrogen and oxygen; e.g., pressurized air may be used. It is also presently believed that the use of carbon dioxide as propellant could be especially advantageous from the point of view of promoting relatively greater penetration of the oxygen through the skin layers. The person skilled in the art will be aware that using carbon dioxide in an aqueous medium will create an acidic environment, and will be able to assess the desirablity or otherwise of using carbon dioxide as a propellant, taking all relevant factors into consideration.

Alternatively, there may be used a propellant which comprises at least one member selected from the group consisting of propane, butane, isobutane, trichloromonofluoromethane, dichlorodifluoromethane, dichlorotetrafluoroethane, monobromomonochlorodifluoromethane, monobromotrifluoromethane, trichlorotrifluoroethane, tetrafluoroethane, octafluorocyclobutane and dimethyl ether. Higher-boiling materials such as (e.g.) pentane and hexane which would be unsuitable for use as propellants on their own, could possibly used as ingredients of propellant mixtures with lower-boiling components. In view of the flammability of hydrocarbon propellants and of dimethyl ether, and their explosive potentiality when admixed with oxygen, the usual precautions should be exercised when using these materials. Where there is no objection to the use of fluorocarbons (which are usually non-flammable) as propellants, it is particularly preferred that such propellant comprises a mixture of dichlorodifluoromethane and dichlorotetrafluoroethane. Insofar as certain countries, states and local authorities may have enacted legislation prohibiting the use of at least certain of the fluorinated hydrocarbons as propellants due to a belief that they may cause irrevocable damage to the ozone layer, the present disclosure is not intended to invite use of such propellants contrary to this legislation. Indeed, the attention of the skilled person is directed to the alternative propellants which may be used in accordance with the invention.

It will be appreciated that propellants about which concern has been expressed with regard to potential damage to the ozone layer are perhalogenated, i.e. fully halogenated, fluorinated hydrocarbons. Consequently there is at the present time a continuing interest in fluorinated hydrocarbon propellants which are not fully halogenated, such as the following:

1,1,1,2-tetrafluoroethane (134A) - this will probably be stable in all emulsions and aqueous solutions, avoiding possible hydrolysis problems;

1,1-dichloro-2,2,2-trifluoroethane (123) appears to be stable in acid media;

difluoromonochloromethane (22) is until now the most widely used propellant which replaces fully halogenated fluorocarbons, but is relatively unstable in aqueous media and moreover has a high pressure of about 8.8 bar at 21°C;

1-chloro-1,1-difluoroethane (142B) is relatively unstable in aqueous media at pH above about 10, and also has a tendency to flammability.

It is presently believed that mixtures of (i) 134A with (ii) either 123 or <70% 142B, and optionally (iii) 22, would provide a propellant with a similar pressure profile to the propellants being currently used. It is also believed to be a distinct possibility that the problem of the relative instability in aqueous media of the newer fluorocarbon propellants may be effectively countered by the presence in these media of epoxides such as propylene oxide, or epoxidized oils such as Epoxol 9-5 (Swift Technical Products), which is stated to be an efficient scavenger of halogen and mineral acids.

A further hazard in the case of ethers such as dimethyl ether, is the possibility of forming explosive peroxides; therefore, such propellants should not be used in the absence of effective peroxide formation inhibitors. As is known in the aerosol art, it is undesirable to use trichloromonofluoromethane as a propellant for aqueous media, because of its liability to hydrolysis; however, this substance may often be suitable for use in the case of substantially non-aqueous carrier media, such as those comprising alcohols and/or paraffin oil.

Many alternatives in the use of pressurized gas propellants will suggest themselves to those skilled in the art. In one alternative, already indicated above, an aerosol canister containing a skin treatment composition (including foam stabilizer) may be pressurized with oxygen, which thus acts as the active ingredient as well as propellant. Alternatively, an optimum predetermined amount of oxygen may be added so as to be substantially completely dissolved in the composition, and the canister may then be pressurized with e.g. compressed air or carbon dioxide. In another alternative, a re-usable high pressure vessel containing the composition components including a foam stabilizer and oxygen may be pressurized with gas added from an attachable reservoir such as a cartridge; in this case, pressurized propellant gas may be released into the vessel (by for example piercing a valve in the neck of a cartridge) when the reservoir is (e.g.) screwed in, as is known for example, in the case of domestic devices for making carbonated water where the carbon dioxide is injected into the contents of a pressure bottle in this manner.

As regards the proportions of the ingredients of the admixture, it is preferred that this comprises in general about 0.25 to about 2.0 wt.% molecular oxygen and about 98.0 to about 99.75 wt.% carrier. When using an aerosol or other pressure vessel, the mixture of ingredients therein may comprise, e.g., about 0.25 to about 2.0 wt.% molecular oxygen, about 1.0 to about 25.0 wt.% propellant, about 72.0 to about 97.75 wt.% carrier, and about 0.05 to about 1.0 wt.% foam stabilizer; and preferably about 1.25 to about 1.35 wt.% molecular oxygen, about 9.0 to about 11.0 wt.% propellant, about 86.65 to about 89.6 wt.% carrier, and about 0.05 to about 1.0 wt.% foam stabilizer.

The invention will now be illustrated by the following Examples, in which the commercially available body lotion specified incorporates foam stabilizer.

## EXAMPLE I

In accordance with substantially known industrial procedures, a standard aerosol can of approximately 200 c.c. capacity was filled with an admixture of 90 g. commercially available body lotion, a propellant which comprised dichlorodifluoromethane (4 g.) and dichlorotetrafluoroethane (6 g.). and 1.3 g. molecular oxygen. The initial pressure of the can prior to addition of oxygen was about 40 p.s.i.g. at 70°F.

The contents of the aerosol can were well shaken and the admixture contained therein, which was propelled therefrom in the form of a stabilized foam, was applied to newborn hairless mice on the dorsal side. On each mouse, the area of application was divided into two sections, to one of which was applied the admixture in accordance with the invention, and to the other of which was applied the same body lotion without admixed oxygen. After two hours, the mice were sacrificed, the skin was peeled and mitochondria were isolated according to the method described by T.P. Singer in "Methods of Biochemical Analysis" (Ed. D. Glick) 22: 123-75 (1974). The activity of two of the main components of the respiratory chain, succinic oxidase and NADH oxidase, were assayed according to Singer's method. The results are summarized in the following table, in which the data indicate that due to the presence of oxygen in the lotion, the rate of activity of essential components of the respiratory chain of the cells is significantly elevated, thus demonstrating an enhancement of the general respiratory metabolism of the cells.

| Body lotion | Enzyme specific activity | |
|---|---|---|
| | succinic oxidase | NADH oxidase |
| control | 131.5 | 64.8 |
| + oxygen | 182.4 | 103.0 |

The control experiment related to a foam generated in the presence of air, and was very much less effective than the experiment of the invention generated with molecular oxygen.

## EXAMPLE II

The following composition may be used in place of the admixture of Example I, and gives similar results, namely, 95 g. commercially available body lotion, a propellant which comprised butane (3.75 g.) and propane (1.25 g.), and 1.3 g. molecular oxygen. The initial pressure of the can prior to addition of oxygen was about 40 p.s.i.g. at 70° F.

## EXAMPLE III

The following composition may be used in place of the admixture of Example II, and gives similar results, namely, 95 g. commercially available body lotion, a propellant which comprised butane (3.33 g.), isobutane (0.5 g.) and propane (1.17 g.), and 1.3 g. molecular oxygen. The initial pressure of the can prior to addition of oxygen was about 40 p.s.i.g. at 70° F.

## EXAMPLE IV

An aerosol can which contained 100 g. commercially available body lotion was pressurized with oxygen only, to a pressure of 9 atm. gauge at 70° F. In this instance, the oxygen served the dual function of dissolving as active ingredient in the body lotion, as well as a propellant. In place of an aerosol can, there may be utilized a re-usable pressure vessel to which the oxygen may be added by an attachable reservoir.

## EXAMPLE V

A mixture of commercially available body lotion (100 g.) with molecular oxygen (1.3 g.) in an aerosol container under slight superatmospheric pressure is subsequently pressurized to 9 atm. gauge at 70° F using as propellant nitrogen, compressed air or carbon dioxide. In place of an aerosol can, there may be utilized a re-usable pressure vessel to which the propellant may be added by an attachable reservoir.

While the invention has been particularly described and exemplified with respect to certain embodiments, it will be apparent to those skilled in the art that modifications and variations may be made in different aspects of the invention, as for example, in the selection of the carrier, in the proportions of the ingredients, and in the nature and composition of the propellant, where used. Accordingly, the invention is not to be construed as limited to such embodiments, rather it will be defined only in accordance with the claims which follow.

**Claims**

1. A composition of matter for promoting skin health, which comprises a physical admixture of:

(A) at least about 0.25 wt.% and preferably no more than about 2.0 wt.% molecular oxygen, with

(B) no more than about 99.75 wt.% and preferably no less than about 98.0 wt.% of a dermatologically acceptable, preferably cosmetically acceptable, fluorocarbon-free carrier; and optionally

(C) an antioxidant;

the composition being maintained under superatmospheric pressure in a pressurized container.

2. A composition according to claim 1, wherein the carrier comprises at least one member selected from the group consisting of water, ethanol, propanol, isopropanol, glycols, esters and paraffin oil.

3. A composition according to either claim 1 or claim 2, wherein water is substantially absent from the carrier.

4. A composition according to any of the preceding claims, wherein component (C) is present and comprises at least one member selected from the group consisting of BHA (butylated hydroxy anisole), BHT (butylated hydroxy toluene), beta-carotene, propyl gallate and alpha-tocopherol.

5. A composition of matter for promoting skin health, which comprises a physical admixture of:

(A) at least about 0.25 wt.%, based on the overall composition, of molecular oxygen, with

(B) a dermatologically acceptable, preferably cosmetically acceptable, carrier; and optionally

(C) an antioxidant;

the composition being maintained under superatmospheric pressure in a pressurized container, and the following ingredient also necessarily being present, namely:

(D) a propellant; and

(E) a foam stabilizer;

and provided that when said propellant comprises molecular oxygen, then said carrier is a fluorocarbon-free carrier.

6. A composition according to claim 5, wherein the propellant comprises at least one component selected from the group consisting of air, carbon dioxide, nitrous oxide, argon, nitrogen and oxygen.

7. A composition according to claim 5, wherein the propellant comprises at least one member selected from the group consisting of propane, butane, isobutane, trichloromonofluoromethane, dichlorodifluoromethane, difluoromonochloromethane, difluoromonochloroethane, dichlorotetrafluoroethane, dichlorotrifluoroethane, monobromomonochlorodifluoromethane, monobromotrifluoromethane, trichlorotrifluoroethane, tetrafluoroethane, octafluorocyclobutane, and dimethyl ether in presence of a peroxide formation inhibitor.

8. A composition according to claim 7, wherein the propellant comprises at least one member selected from the group consisting of 1,1,1,2-tetrafluoroethane, 1,1-dichloro-2,2,2-trifluoroethane, difluoromonochloromethane and 1-chloro-1,1-difluoroethane.

9. A composition according to claim 8, wherein the composition includes a scavenger for at least one member of the group consisting of halogen and mineral acids.

10. A composition according to claim 7 or claim 8, wherein the propellant comprises trichloromonofluoromethane, and the carrier is a substantially non-aqueous carrier.

11. A composition according to any of claims 5 to 10, wherein said physical admixture comprises about 0.25 to about 2.0 wt. % molecular oxygen, about 1.0 to about 25.0 wt. % propellant, about 72.0 to about 97.75 wt. % carrier and about 0.05 to about 1.0 wt. % foam stabilizer.

12. A method for promoting skin health to improve the cosmetic appearance of the skin, which comprises applying to the skin of a living human a composition of matter according to any of the preceding claims.

13. A process for the preparation of a composition according to any one of claims 1 to 11 which comprises admixing at least about 0.25 wt. %, based on the overall composition, of molecular oxygen, and a dermatologically acceptable carrier, and packaging the composition under superatmospheric pressure in a container.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-1 271 181 (ENTREMONT et al.)<br>* Whole document *<br>--- | 1-2,5-6,12-13 | A 61 K 7/48<br>A 61 K 7/00 |
| A,D | FR-A-2 280 360 (SOCIETE GENERALE DE RECHERCHES ET D'APPLICATIONS SCIENTIFIQUES)<br>* Whole document *<br>--- | 1-13 | |
| A | PATENT ABSTRACTS OF JAPAN, vol. 11, no. 300 (C-449)[2747], 29th September 1987;<br>& JP-A-62 93 211 (SHISEIDO CO. LTD) 28-04-1987<br>* Abstract *<br>--- | 1-13 | |
| A | GB-A- 403 062 (LASZLO)<br>* Whole document *<br>--- | 1-13 | |
| A | US-A-3 947 568 (BATES et al.)<br>* Whole document *<br>------ | 1-13 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 11-10-1989 | FISCHER J.P. |